# EUROPÄISCHE PATENTSCHRIFT

(11) **EP 3 657 941 B1**
(45) Veröffentlichungstag und Bekanntmachung des Hinweises auf die Patenterteilung: **16.03.2022**
(21) Anmeldenummer: 18746172.8
(22) Anmeldetag: 25.07.2018
(51) Int. Cl.: A01M 1/02, A01M 1/06, A01M 1/08

(54) **VERFAHREN UND SYSTEM ZUR ERFASSUNG UND/ODER ÜBERWACHUNG VON POPULATIONEN VON INSEKTEN**
METHOD AND SYSTEM FOR RECORDING AND/OR MONITORING POPULATIONS OF INSECTS
PROCÉDÉ ET SYSTÈME POUR L'ENREGISTREMENT ET/OU LA SURVEILLANCE DE POPULATIONS D'INSECTES

(30) Priorität: 28.07.2017 DE 102017213076
(43) Veröffentlichungstag der Anmeldung: 03.06.2020
(73) Patentinhaber: Biogents Aktiengesellschaft, 93055 Regensburg (DE)
(72) Erfinder: GEIER, Martin, 93055 Regensburg (DE); WEBER-GRABAU, Michael, Sunnyvale, CA 94087 (US)
(74) Vertreter: Benninger, Johannes
(86) Internationale Anmeldenummer: PCT/EP2018/070179
(87) Internationale Veröffentlichungsnummer: WO 2019/020694

(56) Entgegenhaltungen:
- WO-A1-2012/054397
- WO-A1-2016/168347
- US-A1- 2003 184 442
- US-A1- 2005 018 745
- US-A1- 2009 223 115

## Beschreibung

Die vorliegende Erfindung betrifft ein Verfahren zur Erfassung und/oder Überwachung von Populationen von Insekten mit den Merkmalen des unabhängigen Verfahrensanspruchs 1. Zudem betrifft die Erfindung ein Insektenerfassungs- und/oder Überwachungssystem zur Erfassung und/oder Überwachung von angelockten und zumindest temporär innerhalb eines definierten Raumes festgehaltenen sowie in Bezug auf definierbare Merkmale sensierten und/oder analysierten Insekten oder Fluginsekten wenigstens einer definierten Gattung mit den Merkmalen des unabhängigen Anspruchs 17.

Zur Überwachung von Maßnahmen zur Insektenbekämpfung sowie auch zur Überprüfung der Wirksamkeit der Maßnahmen sind zahlreiche unterschiedliche Verfahren verfügbar und anwendbar. Insgesamt besteht ein weltweit steigendes Interesse an Verfahren zur Bestandskontrolle und insbesondere zur Reduzierung von bestehenden Populationen von Schadinsekten sowie von solchen Populationen, die als Krankheitsüberträger bekannt sind. Eine Möglichkeit zur Reduzierung und Zurückdrängung solcher Populationen besteht in der Freisetzung von unfruchtbaren Insekten innerhalb existierender Bestände, was auch als sog. "sterile insect technology" (SIT) bekannt ist. Ein besonderes Augenmerk liegt hierbei auf einer bestimmten Spezies von Moskitos, den Aedes Aegypti, die als besonders problematische Art bei der Verbreitung des Zika-Virus in sich derzeit ausbreitenden Epidemien anzusehen ist, jedoch ebenso für Dengue-Epidemien, die Verbreitung von Gelbfieber und anderen Krankheiten verantwortlich gemacht wird.

Die mancherorts bereits eingesetzte Technik des SIT beruht auf der Freisetzung von unfruchtbaren männlichen Moskitos, die in Laboren gezüchtet wurden. Diese männlichen Insekten der zurückzudrängenden Spezies werden in großer Zahl freigesetzt, damit sie sich mit wilden weiblichen Insekten paaren können und hierbei insbesondere in Konkurrenz mit freilebenden männlichen Insekten treten. Als Ergebnis dieser Technik reduziert sich die solcherart beeinflusste Population, womit auch das Risiko der Übertragung von Krankheiten zurückgeht.

Bei einem anderen, jedoch vom Prinzip her ähnlichen Ansatz, werden wild lebende Insektenpopulationen mit einem Bakterium namens Wolbachia infiziert, da dieses Bakterium in der Lage ist, bei solchermaßen infizierten Insekten deren Übertragungsvermögen für Denguefieber zu reduzieren.

Allen diesen vergleichbaren Ansätzen ist gemeinsam, dass sie die extensive Überwachung von männlichen und weiblichen Moskitos der betroffenen Populationen erfordern, wobei deren relevante Attribute zuverlässig erkannt werden sollen. Für die Anwendung der SIT-Technik ist es beispielsweise wünschenswert, freigesetzte männliche Insekten, die im Labor gezüchtet wurden, von wildlebenden männlichen Insekten unterscheiden zu können, was etwa mittels Markierungen auf Grundlage fluoreszierenden Staubes erfolgen kann, wodurch die Zählung zuvor freigesetzter und später wieder eingefangener männlicher Insekten ermöglicht ist. Die derzeit verfügbaren und in der Praxis eingesetzten Verfahren für eine solche Überwachung und Erfassung freigesetzter männlicher Insekten, die aus Laborbeständen stammen, beruhen auf einer manuellen Zählung und erfordern einen höhen Überwachungs- und Laboraufwand.

Aus dem Stand der Technik sind verschiedene Ansätze zur Erfassung und Überwachung von Insektenpopulationen bekannt. So offenbart etwa die WO 2012 054 397 A1 eine Insektenüberwachungsvorrichtung mit einem Köder zum Anlocken von Insekten sowie mit einem oder mehreren Sensoren zur Detektion von Insekten, insbesondere einer bestimmten Insekten-Spezies. Als Sensoren können beispielsweise Bioimpedanzsensoren, optische Sensoren wie IR-Sensoren, Ultraschallsensoren oder dergleichen vorgesehen sein. In einigen Ausführungsformen kann mit den optischen Sensoren die Spezies unterschieden werden. Auch ein Zählen der Insekten wird vorgeschlagen. Die Signale der Sensoren können anschließend in einem Netzwerk gesammelt, weiterverarbeitet und protokolliert werden.

Die WO 2012 054 990 A1 offenbart eine Vorrichtung zur Echtzeit-Erfassung von Insekten. Die Vorrichtung umfasst einen offenen Inspektionszylinder mit einem Kamerasystem zur Echtzeit-Insektenüberwachung und zur Datenerfassung. Mit dem Kamerasystem soll es möglich sein, qualitativ hochwertige Bilder der jeweiligen Insekten zu gewinnen sowie die Insekten zu zählen. Insekten innerhalb des Zylinders können darüber hinaus durch einen Infrarotdetektor detektiert werden.

Die WO 2016 064 735 A1 offenbart ein drahtloses Sensorsystem zur Erfassung des Wachstums von Moskitopopulationen sowie zur Datengewinnung und Datenauswertung. Mit dem Sensorsystem soll es möglich sein, die Moskitopopulation zu erfassen, zu überwachen sowie zu steuern. Die Anzahl der Moskitos kann über entsprechende Zählvorrichtungen erfasst werden. Darüber hinaus können weitere Daten, wie Druck, Temperatur, Feuchtigkeit, Bewegung und Zeit etc. berücksichtigt werden. Die Daten werden jeweils an eine Rechnereinheit weitergeleitet und dort entsprechend verarbeitet.

Die WO 2016 168 347 A1 beschreibt ein eine Moskitofalle sowie ein Netzwerk sowie ein Verfahren zum Erfassen, Zählen, Fangen und Verwerfen von Mücken und/oder Insekten. Die Vorrichtung umfasst unter anderem eine oder mehrere Tonerfassungsvorrichtungen, Videoerfassungsvorrichtung und Detektoren, die jeweils so konfiguriert sind, um Art und Geschlecht des jeweiligen Moskitos und Insekts zu detektieren. Weiter können Sensoren, wie IR-Sensoren, Näherungssensoren und Ultraschallsensoren vorgesehen sein, wodurch die Erkennungsgenauigkeit der Moskitos/Insekten erhöht wird. Die Moskitos/Insekten werden über eine sog. Anziehungsvorrichtung angelockt, welche unterschiedlich ausgebildet sein kann.

Die EP 2 149 301 B1 offenbart eine automatisierte Bestimmung der Anwesenheit von Insekten auf einer Fangplatte. Hierzu ist eine Kamera vorgesehen, welche auf die Oberfläche der Fangplatte fokussiert ist. Mit dieser soll es möglich sein, Anzahl und Art der Insekten, zum Beispiel über visuelle Technologien, zu bestimmen. Die Kamera kann vorzugsweise durch einen Scanner ausgebildet sein. Darüber hinaus ist es auch möglich, die Insekten zu untersuchen und beispielsweise durch Farbe erkennbar zu machen. In Abhängigkeit der gewonnenen Daten können Aussagen über die Entwicklung einer Population getroffen werden.

Die US 2015 023 40 49 A1 offenbart darüber hinaus ein Erfassungssystem für Objekte wie Insekten, das ein zu beleuchtendes Gehäuse umfasst, das mit einem Sensor ausgestattet ist, der Helligkeitsschwankungen erfassen und auf diese Weise die Anwesenheit von sich bewegenden Objekten innerhalb des Gehäuses detektieren kann. Das Messprinzip beruht darauf, dass sich bewegende Objekte wie Fluginsekten verschiedene Einflüsse auf das vom Sensor erfasste Licht haben, wie etwa Lichtabsorption, sich verändernde Schattenwürfe und andere Effekte, welche jeweils vom Sensor erfasst werden können. Die Sensordaten können ausgewertet werden, wodurch Informationen über die Anwesenheit und/oder die Art der erfassten Objekte oder Fluginsekten innerhalb des Gehäuses gewonnen werden können.

Das Dokument WO 2016/168347 A1 offenbart weiterhin eine intelligente Moskitound Insektenfallenvorrichtung, sowie Netzwerke und Verfahren zum Zählen einer Population von Moskitos und/oder Insekten. Die Vorrichtung umfasst ein Gehäuse mit einem schmalen Zugang, um ein Insekt oder eine Mücke in eine bestimmte Richtung zu bewegen. Angelockt werden können die Insekten durch eine Anziehungseinrichtung welche in Form von Lichtern, Summern, Vibrationen oder Lautsprechern ausgebildet sein kann und die Insekten etc. in die Einlasskammer lockt. Die Vorrichtung umfasst außerdem einen oder mehrere Detektoren, um ein Insekt entlang des Zugangs zu erfassen. Weitere können im Bereich einer Erfassungskammer angeordnet sein. An einer Stelle unterhalb oder am Ende des Zugangs werden die Insekten in einem Behälter gesammelt. Mittels einer Recheneinrichtung können erfasste Kenndaten der Insekten bestimmt werden.

Mittels des Einsatzes solcher Systeme sind nicht nur Fernüberwachungen in Echtzeit ermöglicht, sondern sie ermöglichen es je nach Signalverarbeitung darüber hinaus, eine Aufzeichnung und Speicherung der erfassten Sensorwerte vorzunehmen, um etwa eine ständige oder in zyklischen Abständen erfolgende Analyse der Prozesse durchzuführen.

Somit besteht ein vorrangiges Ziel der vorliegenden Erfindung in der Entwicklung einer effektiven Methode, die eine automatische Zählung von Insekten wie Moskitos ermöglicht, wobei die Methode nicht nur das Anlocken von männlichen und weiblichen Insekten umfassen soll, sondern auch die die Identifizierung der Insektenarten oder Insektengattungen, die Unterscheidung männlicher von weiblichen Insekten, die Erkennung von Insekten oder Moskitos, die beispielsweise ein definiertes Attribut oder eine definierte Markierung tragen, die Bestimmung der Insektendichte oder Moskitodichte in einem definierbaren Gebiet, die Übertragung der solchermaßen gewonnenen Daten an eine entfernte Datenerfassungs- und/oder Verarbeitungszentrale, sowie die Verarbeitung und Auswertung dieser Daten.

Dieses Ziel wird mit den Gegenständen der unabhängigen Ansprüche erreicht. Merkmale vorteilhafter Weiterbildungen der Erfindung finden sich in den abhängigen Ansprüchen.

Zur Erreichung des genannten Ziels schlägt die Erfindung ein Verfahren zur Erfassung und/oder Überwachung von Populationen von Insekten, bei dem Insekten wenigstens einer definierten Gattung mit den Merkmalen des unabhängigen Verfahrensanspruchs vor. Das Verfahren dient insbesondere der Erfassung und/oder Überwachung von Populationen von Fluginsekten einer definierten Gattung wie etwa Moskitos, Mücken wie Aedes aegypti oder anderen Arten oder Gattungen, indem die Insekten angelockt, zumindest temporär innerhalb eines definierten Raumes festgehalten sowie in Bezug auf definierbare Merkmale sensiert und/oder analysiert werden. Dieses Anlocken meint auch, die Insekten in den Bereich des definierten Raumes zu bringen, indem sie dorthin angelockt werden. Bei dem erfindungsgemäßen Verfahren wird die definierte Art oder Gattung von Insekten oder Fluginsekten in den Bereich oder innerhalb einer definierbaren räumlichen Umgebung mittels für die Insekten oder Fluginsekten attraktiver Lockreize angelockt, wonach die angelockten Insekten oder Fluginsekten in einen definierten Rauminhalt, insbesondere in einen Innenraum einer Insektenfalle unter Verhinderung eines ungewollten Entweichens zumindest eines Großteils der in den definierten Rauminhalt überführten Insekten oder Fluginsekten zumindest für ein definierbares Zeitintervall überführt werden. Das Überführen der Insekten in den definierten Rauminhalt und/oder das Festhalten der Insekten dort kann auf unterschiedlichen Wegen erfolgen, so etwa mittels einer Saugeinrichtung und/oder mittels Elementen einer Saugfalle.

Weiterhin sieht das Verfahren das Erfassen spezifischer Merkmale der innerhalb des definierten Rauminhalts bzw. der Insektenfalle befindlichen Insekten oder Fluginsekten mittels einer dafür geeigneten bzw. ausgestatteten Sensorik sowie das Bereitstellen der mittels der Sensorik erfassten spezifischen Merkmale der Insekten oder Fluginsekten und Übermittlung der von der Sensorik gelieferten Sensorsignale an eine der Sensorik nachgeschaltete elektronische Analyse- und/oder Auswerteeinrichtung vor.

Bei dem Verfahren kann bspw. vorgesehen sein, dass als spezifische Merkmale der innerhalb des definierten Rauminhalts bzw. der Insektenfalle befindlichen Insekten oder Fluginsekten das Geschlecht (m/w) und/oder die Art bzw. Gattung der Insekten erfasst werden. Da sich nur die weiblichen Insekten fortpflanzen, die von den weiblichen Insekten abgelegten Eier jedoch von männlichen Insekten befruchtet werden müssen, hat es sich als wirksame Maßnahme zur Kontrolle von Insektenpopulationen erwiesen, eine Konkurrenzsituation dergestalt herzustellen, dass große Mengen an unfruchtbaren männlichen Insekten der zurückzudrängenden Art oder Gattung ausgesetzt werden, die in Konkurrenz mit den wild vorkommenden männlichen Insekten treten, so dass durch mangelnde Befruchtung der weiblichen Insekten allmählich weniger neue Insekten entstehen können, so dass insgesamt eine Reduzierung der Populationen erreicht werden kann. Wenn der Erfolg solcher Maßnahmen überprüft, überwacht und gesteuert werden soll, ist es notwendig, männliche Insekten von weiblichen Insekten der zu überwachenden Art oder Gattung unterscheiden zu können.

Zu diesem Zweck sieht das Verfahren etwa vor, dass die spezifischen Merkmale der innerhalb des definierten Rauminhalts bzw. der Insektenfalle befindlichen Insekten oder Fluginsekten durch an den Insekten oder Fluginsekten anhaftenden und/oder diesen eingeprägten Markierungen gebildet werden. Wenn unfruchtbare männliche Insekten innerhalb einer bestimmten Umgebung freigesetzt werden, so ist es sinnvoll, diese Insekten mit einer Markierung zu versehen, die mit Hilfe des erfindungsgemäßen Überwachungsverfahrens erkannt und sensiert werden kann. Solche weitgehend unverlierbaren Markierungen sind ein wertvolles Unterscheidungsmerkmal zwischen im Labor gezüchteten und später freigesetzten Insekten gegenüber wildlebenden gleichartigen Insekten, die ansonsten kaum zuverlässig zu unterscheiden wären.

Die Markierungen können bspw. durch gentechnische Veränderungen an den Insekten oder Fluginsekten gebildet sind, die mittels der Sensorik erfasst werden. Wahlweise können die Markierungen auch durch radioaktive Markierungen gebildet sind, die mittels der Sensorik erfasst werden. Ebenso denkbar ist es, dass die Markierungen durch optische und/oder fluoreszierende Markierungen gebildet sind, die mittels der Sensorik erfasst werden. Zu diesem Zweck eignen sich bspw. fluoreszierende Stoffe wie etwa fluoreszierender Staub, der an den Insekten anhaftet und schon in kleinsten Mengen mittels der eingesetzten Sensorik erkannt werden kann. Vorzugswiese werden solche Markierungen für das erfindungsgemäße Verfahren eingesetzt, die durch den erfassten und/oder freigesetzten Populationen aufgeprägte Merkmale und/oder Markierungen gebildet sind, die mittels der Sensorik mit ausreichender Zuverlässigkeit erfasst werden können, so dass z.B. nur männliche, unfruchtbare Insekten ausgesetzt mit den spezifischen Markierungen versehen sind, so dass die spätere Identifizierung von ausgesetzten Insekten und ihre Unterscheidung von Bestandsinsekten eines innerhalb des überwachten Raumes bereits vorhandenen Insekten ermöglicht ist.

Eine weitere sinnvolle Variante einer optischen und/oder auf sonstiger Weise sensorisch erfassbaren Markierung kann bspw. durch die Verwendung von Rhodamin-B gebildet sein. Wenn den zu sensierenden Insekten durch geeignete Fütterung dieses Rhodamin-B verabreicht wurde, etwa durch Einmischen einer geringen Konzentration in geeigneten Futterstoffen wie Zuckerlösung oder Honiglösungen, kann dieses fluoreszierende Färbemittel mittels geeigneter Sensorik an den überwachten Insekten effektiv erfasst werden.

Auch sind Kombinationen solcher Markierungen möglich, um die Zuverlässigkeit der Erkennung zu verbessern. Beim Einsatz einer entsprechend empfindlichen Sensorik können schon die kleinsten Spuren solcher Markierungen genügen, damit eine Unterscheidung der solchermaßen markierten von freifliegenden oder freilebenden, wilden Insekten möglich ist.

Eine vorteilhafte Variante des erfindungsgemäßen Verfahrens kann vorsehen, dass die zur Erfassung der spezifischen Merkmale der innerhalb des definierten Rauminhalts bzw. der Insektenfalle befindlichen Insekten oder Fluginsekten geeignete bzw. ausgestattete Sensorik durch optische Erfassungseinrichtungen gebildet ist oder optische Erfassungseinrichtungen umfasst. Mit optischen Erfassungseinrichtungen sind in diesem Zusammenhang alle Sensoriken gemeint, die in der Lage sind, elektromagnetische Wellen oder Änderungen im Abstrahl- oder Reflexionsverhalten für elektromagnetische Wellen im Infrarotbereich, im sichtbaren Bereich und/oder im ultravioletten Bereich, zu erfassen. Hierbei können die optischen Erfassungseinrichtungen insbesondere eine der Sensorik nachgeschaltete Bildauswertung umfassen, wobei auch die Erfassung reflektierender Elemente oder Bildanteile sinnvoll sein kann. Mittels der optischen Erfassungseinrichtungen und/oder der nachgeschalteten Bildauswertung können wahlweise auch Bewegungsmuster der sensierten Insekten oder Fluginsekten erfasst und/oder der nachgeschalteten elektronischen Analyse- und/oder Auswerteeinrichtung zur Verfügung gestellt werden. Grundsätzlich sind auch jegliche andere Erfassungsvarianten denkbar, bspw. die optische Erfassung akustischer Muster, die von den betreffenden Insekten emittiert werden, oder von Flügelschlagprofilen, die für die betreffenden Insekten charakteristisch sein können. Auch können andere Emissionen erfasst werden, bspw. chemische Stoffzusammensetzungen o. dgl. Wenn in diesem Zusammenhang von einer optischen Erfassung akustischer Muster die Rede ist, so sind damit sog. optoakustische Verfahren gemeint, die bspw. mittels Lasererfassungsmethoden typische akustische Emissionen und Emissionsmuster erfassen können, die von bestimmten Insekten auf jeweils unterschiedliche Weise erzeugt werden, so dass auf Basis einer solchen Mustererfassung und einer nachgeschalteten Auswertung der solchermaßen erfassten Emissionen eine zuverlässige Unterscheidung verschiedener Insektenarten sowie auch eine zuverlässige Unterscheidung männlicher von weiblichen Insekten derselben Art ermöglicht ist.

Sofern die Insekten anhand ihrer akustischen Emissionen unterscheidbar und/oder erkennbar sind, kann das Verfahren wahlweise auch vorsehen, dass die zur Erfassung der spezifischen Merkmale der innerhalb des definierten Rauminhalts bzw. der Insektenfalle befindlichen Insekten oder Fluginsekten geeignete bzw. ausgestattete Sensorik durch akustische Erfassungseinrichtungen gebildet ist oder akustische Erfassungseinrichtungen umfasst. Weitere Varianten des Verfahrens sind denkbar, so bspw., dass die zur Erfassung der spezifischen Merkmale der innerhalb des definierten Rauminhalts bzw. der Insektenfalle befindlichen Insekten oder Fluginsekten geeignete bzw. ausgestattete Sensorik durch für elektromagnetische und/oder radioaktive Strahlung empfindliche Erfassungseinrichtungen gebildet ist oder für elektromagnetische und/oder radioaktive Strahlung empfindliche Erfassungseinrichtungen umfasst.

Das Verfahren kann vorsehen, dass die innerhalb der definierten räumlichen Umgebung oder in der Umgebung einer Insektenfalle angelockten Insekten oder Fluginsekten mittels für die Insekten oder Fluginsekten attraktiver optischer Reize angelockt werden, bspw. mittels Hell-Dunkel-Kontrastflächen o. dgl. Wahlweise kann das Verfahren auch vorsehen, dass die innerhalb der definierten räumlichen Umgebung oder in der Umgebung einer Insektenfalle angelockten Insekten oder Fluginsekten mittels für die Insekten oder Fluginsekten attraktiver akustischer Reize angelockt werden. Auch die Verwendung anderer Reize ist möglich, ebenso die Kombination mehrerer dieser Lockreize. So kann das Verfahren wahlweise auch vorsehen, dass die innerhalb der definierten räumlichen Umgebung oder in der Umgebung einer Insektenfalle angelockten Insekten oder Fluginsekten mittels für die Insekten oder Fluginsekten attraktiver sensorischer Reize und/oder mittels Wärme angelockt werden. Ebenso kann das Verfahren in einer Weise ausgestaltet sein, dass die innerhalb der definierten räumlichen Umgebung oder in der Umgebung einer Insektenfalle angelockten Insekten oder Fluginsekten mittels für die Insekten oder Fluginsekten attraktiver olfaktorischer und/oder chemischer Lockreize angelockt werden.

Eine weitere alternative oder als Ergänzung zu sehende Variante des erfindungsgemäßen Verfahrens kann in einer Weise ausgestaltet sein, dass die innerhalb der definierten räumlichen Umgebung oder in der Umgebung einer Insektenfalle angelockten Insekten oder Fluginsekten mittels für die Insekten oder Fluginsekten attraktiver Lichtreize und/oder mittels für die Insekten oder Fluginsekten attraktiver akustischer Reize angelockt werden. So hat es sich gezeigt, dass Mücken und andere Fluginsekten von Lichtreizen angezogen werden, und dass insbesondere männliche Mücken von ultraviolettem Licht angezogen werden, dieses ultraviolette Licht somit als ausgeprägten Lockreiz empfinden. Darüber hinaus hat sich gezeigt, dass eine Kombination von Lichtreizen, insbesondere unter Verwendung von UV-Licht, mit akustischen Reizen als für die männlichen Insekten besonders attraktiv empfunden wird. Im Speziellen können diese akustischen Reize durch eine Imitation von Flügelschlägen der Insektenweibchen gebildet sein, was von den männlichen Insekten als starker Lockreiz empfunden wird.

Grundsätzlich sollen auch alle Arten von Ansaugeinrichtungen und/oder Elemente von Saugfallen als Lockmittel angesehen werden, da es grundsätzlich auch möglich ist, die Insekten in geeigneter Weise anzusaugen, ohne ihnen spezifische Lockreize zu präsentieren.

Um Doppelzählungen derselben Insekten zu verhindern, kann bei dem Verfahren vorgesehen sein, dass die angelockten Insekten oder Fluginsekten innerhalb des definierten Rauminhalts bzw. im Innenraum der Insektenfalle für ein definiertes Zeitintervall an einer Flucht gehindert werden. So kann das Verfahren in einer Weise ausgestaltet sein, dass die innerhalb des definierten Rauminhalts bzw. im Innenraum der Insektenfalle angelockten Insekten oder Fluginsekten nach ihrer Erfassung wieder freigelassen werden. Die Freilassung nach der Erfassung kann insbesondere dann wichtig und sinnvoll sein, wenn unfruchtbaren männliche Insekten freigesetzt wurden, da diese weiterhin in der untersuchten Umgebung ausschwärmen können, nachdem sie erfasst und wieder freigesetzt wurden.

Alternativ hierzu kann das Verfahren auch in einer Weise abgewandelt werden, dass die angelockten Insekten oder Fluginsekten innerhalb des definierten Rauminhalts bzw. im Innenraum der Insektenfalle für unbestimmte Zeit an einer Flucht gehindert werden. D.h. mit der Falle können die Insekten gefangen, erfasst und anschließend unschädlich gemacht bzw. getötet werden. Auch auf diese Weise kann sichergestellt werden, dass die gefangenen Insekten jeweils nur einmal erfasst werden.

Für die Erfassung der in den Insektenfallen gefangenen Insekten oder Fluginsekten eignen sich bspw. solche Verfahren, die auf einer Erfassung und Vermessung der jeweils in einem Luftstrom getragenen Insekten beruhen. Da diese Verfahren jedoch in der Praxis relativ schwierig zu handhaben sind, kommen auch solche Varianten in Frage, bei denen die Insekten bspw. auf einer definierten Fläche fixiert und dort mittels der Sensorik erfasst werden. Eine solche definierte Fläche kann bspw. eine Klebefläche oder ein Netz o. dgl. sein, die sich innerhalb der Falle befindet, wodurch Mehrfachmessungen zuverlässig zu verhindern sind. , aber ggf. interessant als Merkmal im Anspruch) oder Fangen auf Klebefläche oder auch Netz (Verhinderung von Mehrfachmessungen!) - Insekt im Luftstrom getragen u. wird gleichzeitig vermessen.

Mit dem Verfahren ist es weiterhin möglich, die Sensordaten mindestens einer mit entsprechender Sensorik ausgestatteten Insektenfalle über einen definierten längeren Zeitraum zu erfassen und zur Analyse von Insekten- oder Fluginsektenpopulationen und/oder deren Veränderungen im Zeitverlauf zu erfassen, zu analysieren und aufzubereiten. Vorzugsweise werden jedoch für ein solches Analyseverfahren die Daten mehrerer solcher Fallen genutzt. Die der jeweiligen Sensorik einer jeden einzelnen Falle von insgesamt mehreren solcher Insektenfallen, die ggf. an jeweils unterschiedlichen Orten stehen können, nachgeschaltete elektronische Analyse- und/oder Auswerteeinrichtung kann insbesondere über Datenfernverbindungen mit den Insektenfallen bzw. mit deren Sensoriken in Verbindung stehen, so dass eine zentrale Datenerfassung und -auswertung für ein mit mehreren Fallen, ggf. mit einer Vielzahl von Insektenfallen, ausgestattetes größeres Gebiet ermöglicht ist, ohne dass eine direkte Leitungs- oder Datenverbindung vonnöten ist.

Mit dem erfindungsgemäßen Verfahren können Insekten oder Fluginsekten einer bestimmten Art oder Gattung erfasst und überwacht werden, bspw. Mücken oder Moskitos bestimmter Gattung. Auf diese Weise können Erkenntnisse über Verhältnis der natürlichen Population zu freigelassenen Mücken gewonnen oder auch Daten über die Population allgemein gewonnen werden. So ist bspw. möglich, die Überlebensdauer freigelassener Mücken zu überprüfen, ihre Verteilung, ihr Vorkommen etc. Ein zentraler Aspekt des Verfahrens liegt jedoch in der Unterscheidung von bestimmten Insekten, d.h. insbesondere in der Unterscheidung markierter von nicht markierten Insekten.

Zur Erreichung des oben genannten Ziels schlägt die Erfindung weiterhin ein Insektenerfassungs- und/oder Überwachungssystem zur Erfassung und/oder Überwachung von angelockten und zumindest temporär innerhalb eines definierten Raumes festgehaltenen sowie in Bezug auf definierbare Merkmale sensierten und/oder analysierten Insekten oder Fluginsekten wenigstens einer definierten Gattung mit den Merkmalen des unabhängigen Systemanspruchs vor. Das System umfasst zumindest geeignete Mittel zum Anlocken der definierten Gattung von Insekten oder Fluginsekten innerhalb einer definierbaren räumlichen Umgebung mittels für die Insekten oder Fluginsekten attraktiver Lockreize sowie einen definierten Rauminhalt, gebildet insbesondere durch einen Innenraum einer Insektenfalle, der/die zur Überführung und Aufnahme der angelockten Insekten oder Fluginsekten unter Verhinderung eines ungewollten Entweichens zumindest eines Großteils der in den definierten Rauminhalt überführten Insekten oder Fluginsekten zumindest für ein definierbares Zeitintervall vorgesehen und/oder ausgestattet ist. Weiterhin benötigt das System eine geeignete Sensorik, die zur Erfassung spezifischer Merkmale der innerhalb des definierten Rauminhalts bzw. der Insektenfalle befindlichen Insekten oder Fluginsekten geeignet bzw. ausgestattet ist, und eine der Sensorik nachgeschaltete elektronische Analyse- und/oder Auswerteeinrichtung zur Erfassung und/oder Auswertung der mittels der Sensorik erfassten spezifischen Merkmale der Insekten oder Fluginsekten.

Das erfindungsgemäße System ist insbesondere zur Erfassung von an den Insekten oder Fluginsekten anhaftenden und/oder diesen eingeprägten Markierungen ausgebildet. Die zur Erfassung der spezifischen Merkmale der innerhalb des definierten Rauminhalts bzw. der Insektenfalle befindlichen Insekten oder Fluginsekten geeignete bzw. ausgestattete Sensorik kann bspw. durch optische Erfassungseinrichtungen gebildet sein oder solche optische Erfassungseinrichtungen umfassen. So können die optischen Erfassungseinrichtungen bspw. eine der Sensorik nachgeschaltete Bildauswertung umfassen, wobei auch Einrichtungen zur Erfassung reflektierender Elemente oder Bildanteile sinnvoll eingesetzt werden können. Mittels der optischen Erfassungseinrichtungen und/oder der nachgeschalteten Bildauswertung können wahlweise auch Bewegungsmuster der sensierten Insekten oder Fluginsekten erfasst und/oder der nachgeschalteten elektronischen Analyse- und/oder Auswerteeinrichtung zur Verfügung gestellt werden. Denkbar sind auch Varianten, bei denen vielfältige andere Merkmale erfasst werden, bspw. akustische Muster, Flügelschlagprofile, sonstige Emissionen der Insekten etc. So kann bei dem System die zur Erfassung der spezifischen Merkmale der innerhalb des definierten Rauminhalts bzw. der Insektenfalle befindlichen Insekten oder Fluginsekten geeignete bzw. ausgestattete Sensorik durch akustische Erfassungseinrichtungen gebildet sein oder solche akustische Erfassungseinrichtungen umfassen.

Weiterhin kann das System in einer Weise ausgestattet sein, dass die zur Erfassung der spezifischen Merkmale der innerhalb des definierten Rauminhalts bzw. der Insektenfalle befindlichen Insekten oder Fluginsekten geeignete bzw. ausgestattete Sensorik durch für elektromagnetische und/oder radioaktive Strahlung empfindliche Erfassungseinrichtungen gebildet ist oder für elektromagnetische und/oder radioaktive Strahlung empfindliche Erfassungseinrichtungen umfasst.

Wahlweise kann das System auch dazu in der Lage sein, für die Insekten oder Fluginsekten attraktive optische und/oder akustische und/oder sensorische Reize und/oder Wärmereize und/oder olfaktorische und/oder chemische Lockreize zu verwenden, mit denen die Insekten jeweils anlockbar sind, wobei auch beliebige Kombinationen dieser Lockreizvarianten eingesetzt und angewendet werden können. Wenn von optischen und/oder von akustischen Lockreizen die Rede ist, so ist besonders darauf hinzuweisen, dass Mücken und andere Fluginsekten von Licht angezogen werden, und dass insbesondere männliche Mücken von ultraviolettem Licht angezogen werden und dieses ultraviolette Licht als ausgeprägten Lockreiz empfinden. Darüber hinaus hat sich gezeigt, dass eine Kombination von Lichtreizen, insbesondere unter Verwendung von UV-Licht mit akustischen Reizen als für die männlichen Insekten besonders attraktiv empfunden wird. Im Speziellen können diese akustischen Reize durch eine Imitation von Flügelschlägen der Insektenweibchen gebildet sein, was von den männlichen Insekten als starker Lockreiz empfunden wird.

Der für die Aufnahme angelockter Insekten oder Fluginsekten vorgesehene definierte Rauminhalt bzw. der Innenraum der Insektenfalle kann die aufgenommenen Insekten oder Fluginsekten zumindest für ein definiertes Zeitintervall an einer Flucht hindern. Wahlweise können die Insekten auch unschädlich gemacht werden, nachdem sie erfasst und die erfassten Daten ausgewertet wurden. Die eingesetzten Insektenfallen können insbesondere mit einer Einrichtung zur Fixierung der gefangenen Insekten oder Fluginsekten ausgestattet sein. Eine solche Einrichtung bzw. definierte Fläche in der Falle kann bspw. eine Klebefläche oder ein Netz o. dgl. sein, die sich innerhalb der Falle und im Erfassungsbereich der eingesetzten Sensorik befindet, wodurch Mehrfachmessungen zuverlässig zu verhindern sind. Für die Erfassung der in den Insektenfallen gefangenen Insekten oder Fluginsekten eignen sich bspw. solche Verfahren, die auf einer Erfassung und Vermessung der jeweils in einem Luftstrom getragenen Insekten beruhen, wobei zu erwähnen ist, dass solche Verfahren in der Praxis mehr Aufwand benötigen als solche, bei denen die Insekten fixiert und dort mittels der Sensorik erfasst werden.

Das System kann insbesondere zur Durchführung eines Verfahrens gemäß einer der zuvor beschriebenen Ausführungsvarianten verwendet oder eingesetzt werden.

Es sei an dieser Stelle ausdrücklich erwähnt, dass alle Aspekte und Ausführungsvarianten, die im Zusammenhang mit dem erfindungsgemäßen Insektenerfassungs- und/oder Überwachungssystem erläutert wurden, gleichermaßen Teilaspekte des erfindungsgemäßen Verfahrens betreffen oder sein können. Wenn daher an einer Stelle bei der Beschreibung oder auch bei den Anspruchsdefinitionen zum erfindungsgemäßen Insektenerfassungs- und/oder Überwachungssystem von bestimmten Aspekten und/oder Zusammenhängen und/oder Wirkungen die Rede ist, so gilt dies gleichermaßen für das erfindungsgemäße Verfahren. In umgekehrter Weise gilt dasselbe, so dass auch alle Aspekte und Ausführungsvarianten, die im Zusammenhang mit dem erfindungsgemäßen Verfahren erläutert wurden, gleichermaßen Teilaspekte des erfindungsgemäßen Insektenerfassungs- und/oder Überwachungssystem betreffen oder sein können. Wenn daher an einer Stelle bei der Beschreibung oder auch bei den Anspruchsdefinitionen zum erfindungsgemäßen Verfahren von bestimmten Aspekten und/oder Zusammenhängen und/oder Wirkungen die Rede ist, so gilt dies gleichermaßen für das erfindungsgemäße Insektenerfassungs- und/oder Überwachungssystem.

Im Folgenden sollen Ausführungsbeispiele die Erfindung und ihre Vorteile anhand der beigefügten Figuren näher erläutern. Die Größenverhältnisse der einzelnen Elemente zueinander in den Figuren entsprechen nicht immer den realen Größenverhältnissen, da einige Formen vereinfacht und andere Formen zur besseren Veranschaulichung vergrößert im Verhältnis zu anderen Elementen dargestellt sind.

Fig. 1 zeigt eine schematische Darstellung einer Ausführungsvariante eines erfindungsgemäßen Insektenerfassungs- und/oder Überwachungssystems.

Fig. 2 zeigt eine schematische Darstellung der Wirkprinzipien des Systems gemäß Fig. 1, das zur Durchführung eines erfindungsgemäßen Verfahrens zur Erfassung und/oder Überwachung von Populationen von Insekten einer definierten Gattung vorgesehen bzw. geeignet ist.

Für gleiche oder gleich wirkende Elemente der Erfindung werden identische Bezugszeichen verwendet. Ferner werden der Übersicht halber nur Bezugszeichen in den einzelnen Figuren dargestellt, die für die Beschreibung der jeweiligen Figur erforderlich sind. Die dargestellte Ausführungsform stellt lediglich ein Beispiel dar, wie das erfindungsgemäße optische Überwachungssystem ausgestaltet sein kann, soll jedoch nicht als abschließende Begrenzung aufgefasst werden.

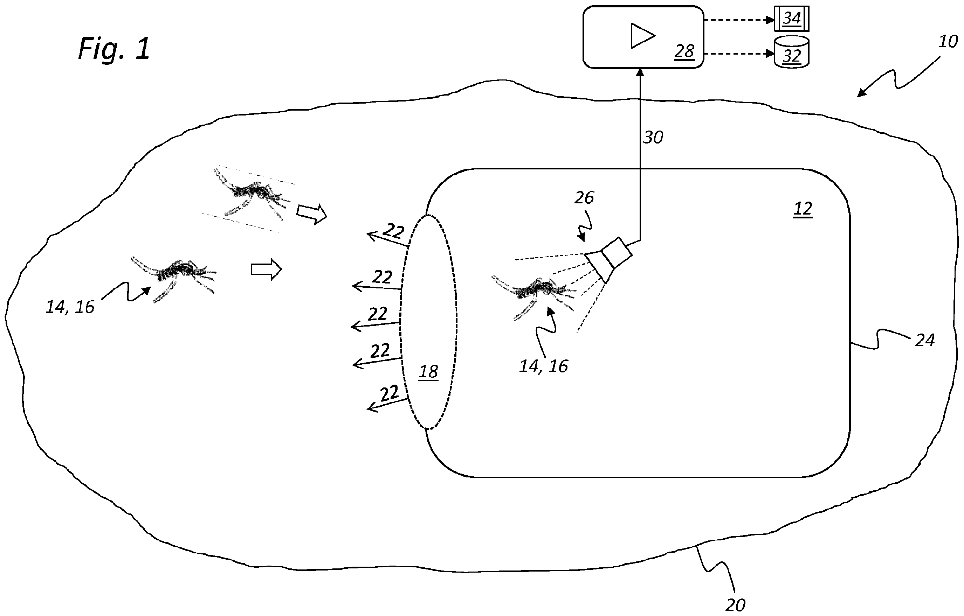

Die schematische Darstellung der Fig. 1 zeigt die wesentlichen Prinzipien einer möglichen Ausführungsvariante eines erfindungsgemäßen Insektenerfassungs- und/oder Überwachungssystems 10 bzw. eines Systems 10 zur Erfassung und/oder Überwachung von angelockten und zumindest temporär innerhalb eines definierten Raumes 12 festgehaltenen sowie in Bezug auf definierbare Merkmale 14 sensierten und/oder analysierten Insekten oder Fluginsekten 16 wenigstens einer definierten Gattung. Die Fluginsekten 16 können bspw. Gelbfiebermücken (Aedes Aegypti) sein, die in manchen Gegenden in großer Zahl vorkommen und deren Bestand mittels des gezeigten Systems 10 überwacht werden kann, sofern die innerhalb des definierten Raumes 12 befindlichen und dort erfassten Insekten 16 über eine eindeutig sensierbare Markierung verfügen, die die definierbaren Merkmale 14 repräsentiert. Aber die Fluginsekten 16 im Sinne der vorliegenden Erfindung können auch nahezu beliebige andere Arten sein, da sich die erfindungsgemäßen Prinzipien bei den unterschiedlichsten Insektenarten anwenden lassen.

Das System 10 umfasst hier zunächst nicht näher bezeichnete Mittel 18 zum Anlocken der definierten Gattung von Insekten oder Fluginsekten 16 innerhalb einer definierbaren räumlichen Umgebung 20, innerhalb derer die Mittel 18 wirksam sind und einen anlockenden Einfluss auf solche Insekten oder Fluginsekten 16 haben, die sich innerhalb der definierbaren räumlichen Umgebung 20 aufhalten. Die Anlockmittel 18 können insbesondere für die Insekten oder Fluginsekten 16 attraktive Lockreize 22 aussenden, die diesen als optische, akustische, olfaktorische oder sonstige Lockreize 22 bspw. eine attraktive Nahrungsquelle signalisieren können. Der definierte Rauminhalt 12, dem die Mittel 18 zum Anlocken der Fluginsekten 16 zugeordnet sind, kann insbesondere durch einen Innenraum einer Insektenfalle 24 gebildet sein, die der Überführung und Aufnahme der angelockten Insekten oder Fluginsekten 16 und der Verhinderung eines ungewollten Entweichens zumindest eines Großteils der in den definierten Rauminhalt 12 überführten Insekten oder Fluginsekten 16 zumindest für ein definierbares Zeitintervall dient.

Die hierbei eingesetzten Insektenfallen 24 können von unterschiedlichster Art sein, je nachdem, welche Insekten 16 angelockt und gefangen werden sollen. Für Gelbfiebermücken oder Moskitos haben sich bspw. Fallen als besonders effektiv erwiesen, die optische Kontrastflächen mit schwachen Lustströmungen kombinieren, was von den betreffenden Insekten als ausgeprägte Lockreize empfunden werden. Eine solche Insektenfalle 24 ist etwa in der WO 2004 054 358 A2 offenbart. Dort ist eine helle Fläche mit einer Ausströmfläche zur Erzeugung einer von der Fläche ausgehenden schwachen Luftströmung kombiniert, wobei sich innerhalb der hellen Fläche mindestens eine dunkle Kontrastfläche befindet. Die dunkle Kontrastfläche kann insbesondere als Ansaugkanal zum Einsaugen der angelockten Fluginsekten 16 ausgebildet sein.

Um seine vorgesehene Aufgabe erfüllen zu können, umfasst das System 10 weiterhin eine geeignete Sensorik 26, die insbesondere innerhalb des Rauminhalts 12 der Falle 24 angeordnet ist, und die für die Erfassung der zuvor definierten spezifischen Merkmale 14 der innerhalb des definierten Rauminhalts 12 bzw. der Insektenfalle 24 befindlichen Insekten oder Fluginsekten 16 geeignet und ausgestattet ist. Allerdings ist zu betonen, dass die Sensorik 26 keineswegs zwingend der Falle 24 räumlich in einer solchen Weise zugeordnet sein muss, dass sie sich in der Insektenfalle 24 befindet. Ebenso kann die Sensorik 26 auch nur in räumlicher Nähe zum definierten Rauminhalt 12 bzw. zur Insektenfalle 24 angeordnet sein. So haben sich bspw. Anordnungen bewährt, bei denen mittels einer optischen Erfassungseinrichtung von oben in die Falle 24 hineingeschaut wird, wobei die Sensorik 26 der dabei eingesetzten optischen Erfassungseinrichtungen nicht unmittelbarer Bestandteil der Insektenfalle 24 ist. Weiterhin ist der Sensorik 26 eine elektronische Analyse- und/oder Auswerteeinrichtung 28 zur Erfassung und/oder Auswertung der mittels der Sensorik 26 erfassten spezifischen Merkmale 14 der Insekten oder Fluginsekten 16 nachgeschaltet, welche die von der Sensorik 26 gelieferten elektronischen Sensorsignale 30 in geeigneter Weise verarbeiten und aufbereiten kann. Vorzugsweise kann die elektronische Analyse- und/oder Auswerteeinrichtung 28 die aus der Verarbeitung und Aufbereitung der von der Sensorik 26 gelieferten Sensorsignale 30 gewonnenen und errechneten Daten in geeigneter Weise speichern und/oder visualisieren bzw. an entfernte Orte übertragen, was durch die mit der Analyse- und/oder Auswerteeinrichtung 28 gekoppelte und als weitere Ausstattungsoption zu verstehende Speichereinrichtung 32 sowie durch die ebenfalls mit der Analyse- und/oder Auswerteeinrichtung 28 gekoppelte und als weitere Ausstattungsoption zu verstehende Anzeigeeinrichtung 34 angedeutet ist.

Wenn in diesem Zusammenhang von einer der Sensorik 26 nachgeschalteten Einrichtung 28 die Rede ist, so ist dies in einem allgemeinen und umfassenden Sinne zu verstehen. So können zahlreiche Sensoriken 26 ihre Daten 30 an eine zentrale Analyseund/oder Auswerteeinrichtung 28 übertragen, ohne dass hierbei eine räumliche Nähe zwischen den Fallen 24 und der Einrichtung 28 gegeben sein muss. Das erfindungsgemäße System 10 kann bspw. auch ein Netzwerk aus mehreren oder vielen Insektenfallen 24 umfassen, die ein größeres Gebiet abdecken können. Die von den Sensoriken 26 der mehreren oder zahlreichen Insektenfallen 24 gelieferten Sensordaten 30 können hierbei bspw. mittels Fernverbindungen (z.B. Funkverbindungen) an die zentrale Datenverarbeitungsanlage 28 geliefert werden, die wahlweise entfernt von den Fallen 24 stehen kann. Die der jeweiligen Sensorik 26 einer jeden einzelnen Falle 24 von insgesamt mehreren solcher Insektenfallen 24, die ggf. an jeweils unterschiedlichen Orten stehen können, nachgeschaltete elektronische Analyse- und/oder Auswerteeinrichtung 28 kann insbesondere über beliebige Datenfernverbindungen mit den Insektenfallen 24 bzw. mit deren Sensoriken 26 in Verbindung stehen, so dass eine zentrale Datenerfassung und Datenauswertung für ein mit mehreren Fallen 24, ggf. mit einer Vielzahl von Insektenfallen 24, ausgestattetes größeres Gebiet ermöglicht ist, ohne dass eine direkte Leitungs- oder Datenverbindung vonnöten ist. Auf diese Weise lassen sich sehr leistungsfähige Monitoring-Systeme für große Gebiete realisieren, die zentral verwaltet werden und mit einer zentralen Datenerfassung und -auswertung ausgestattet sein können.

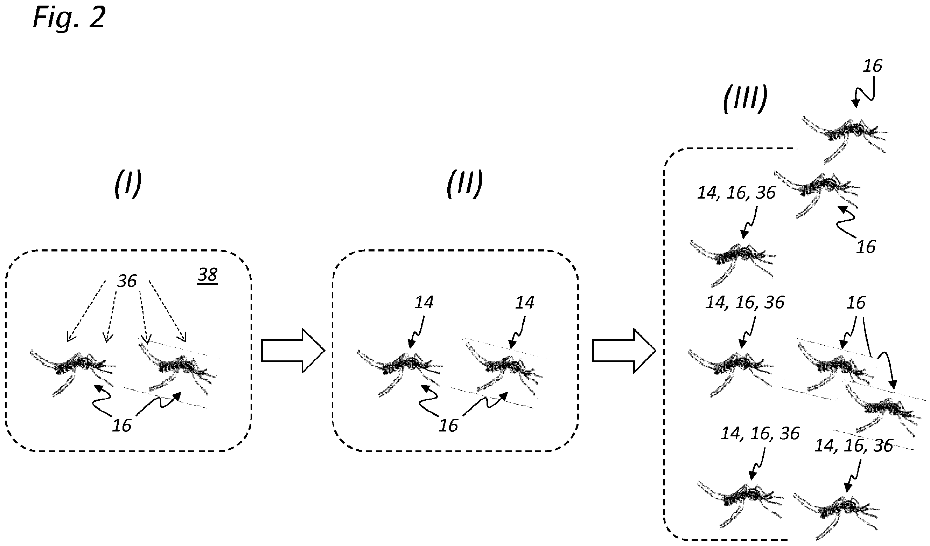

Die schematische Darstellung der Fig. 2 soll die Prinzipien verdeutlichen, die dem in Fig. 1 erläuterten erfindungsgemäßen System 10 zugrunde liegen. Bei dem mit der vorliegenden Erfindung beschriebenen Verfahren handelt es in erster Linie um eine Wirksamkeitsüberwachungsmaßnahme, d.h. es soll die Effektivität und Reichweite von Manipulationsmaßnahmen überprüft und ggf. über längere Zeiträume begleitet und überwacht werden. Wenn in diesem Zusammenhang von bestimmten Merkmalen 14 die Rede ist, die den angelockten und mittels der Sensorik 26 erfassten Fluginsekten 16 anhaften, so können dies bspw. farbige, fluoreszierende oder sonstige optisch erkennbare Markierungen 36 sein, die Fluginsekten 16 zugeführt oder auf diesen aufgebracht werden, die in einem Labor 38 gezüchtet wurden. Es handelt sich bei diesen im Labor 38 gezüchteten Fluginsekten (z.B. Gelbfiebermücken - Aedes Aegypti o.ä.) um unfruchtbare männliche Tiere, die ohne die in Form der Markierungen 36 aufgebrachten sensierbaren Merkmale 14 nach ihrer Freisetzung nicht mehr von wildlebenden Tieren unterscheidbar wären. Nach der Züchtung der Tiere 16 im Labor 38 und der Aufbringung der Markierungen 36 (Phase I - linker Kasten) handelt es sich um freisetzbare männliche und unfruchtbare Fluginsekten 16, die mit unverwechselbaren und eindeutig sensierbaren Merkmalen 14 versehen sind (Phase II - Kasten rechts daneben). Die Markierungen 36 können den Insekten 16 bspw. durch geeignete Fütterung verabreicht und aufgeprägt werden, etwa indem die Insekten 16 mit einer gering konzentrierten Lösung des fluoreszierenden Stoffes Rhodamin-B gefüttert werden, der in gewünschter Weise mittels optischer Sensoren an den Insekten 16 erkannt werden kann.

Die solchermaßen markierten, im Labor 38 gezüchteten unfruchtbaren männlichen Fluginsekten 16 mit den aufgebrachten Markierungen 36 können anschließend innerhalb eines Bestandes von wildlebenden Fluginsekten 16 - dies sind weibliche und männliche Tiere ohne aufgebrachte Merkmale oder Markierungen - freigesetzt werden, was durch den rechten Kasten in Fig. 2 (Phase III) angedeutet ist. Da sich die im Labor 38 gezüchteten Tiere in der bestehenden Population aufhalten und sich mit weiblichen Tieren paaren, jedoch aufgrund ihrer Unfruchtbarkeit deren Fortpflanzung verhindern, wird der bestehende wildlebende Bestand der solchermaßen manipulierten Fluginsekten 16 allmählich reduziert, sofern die Insekten 16 sich so verhalten, wie gewünscht und prognostiziert.

Da jedoch weder die Populationsdichte eines bestehenden Bestandes an wildlebenden Fluginsekten 16 exakt bestimmbar ist, noch die Beeinflussung des Bestandes durch die Freisetzung der markierten unfruchtbaren Insekten 16 durch bloße Beobachtung überprüfbar ist, kann das in Fig. 1 in schematischer Weise beschriebene System 10 dazu dienen, die Bestandsveränderungen zu erfassen und zu dokumentieren, indem die markierten Insekten 16 eindeutig erfasst werden und von nicht markierten, d.h. von wildlebenden Insekten 16 unterschieden werden können.

Die in Fig. 1 gezeigte und zur Erfassung der spezifischen Merkmale 14 der innerhalb des definierten Rauminhalts 12 bzw. der Insektenfalle 24 befindlichen Insekten 16 oder Fluginsekten 16 geeignete bzw. ausgestattete Sensorik 26 kann bspw. durch optische Erfassungseinrichtungen wie etwa Kameras gebildet sein oder solche optische Erfassungseinrichtungen umfassen. So können die optischen Erfassungseinrichtungen bspw. eine der Sensorik 26 nachgeschaltete Bildauswertung (insbesondere innerhalb der elektronischen Analyse- und/oder Auswerteeinrichtung 28) umfassen, wobei auch Einrichtungen zur Erfassung reflektierender Elemente oder Bildanteile sinnvoll eingesetzt werden können. Mittels der optischen Erfassungseinrichtungen und/oder der nachgeschalteten Bildauswertung können wahlweise auch Bewegungsmuster der sensierten Insekten oder Fluginsekten 16 erfasst und/oder der nachgeschalteten elektronischen Analyse- und/oder Auswerteeinrichtung 28 zur Verfügung gestellt werden. Denkbar sind auch Varianten, bei denen vielfältige andere Merkmale erfasst werden, bspw. akustische Muster, Flügelschlagprofile, sonstige Emissionen der Insekten 16 etc. So kann bei dem System 10 die zur Erfassung der spezifischen Merkmale 14 der innerhalb des definierten Rauminhalts 12 bzw. der Insektenfalle 24 befindlichen Insekten 16 oder Fluginsekten 16 geeignete bzw. ausgestattete Sensorik 26 auch durch akustische Erfassungseinrichtungen gebildet sein oder solche akustische Erfassungseinrichtungen umfassen. Wahlweise kann das System 10 auch in einer Weise ausgestattet sein, dass die zur Erfassung der spezifischen Merkmale 14 der innerhalb des definierten Rauminhalts 12 bzw. der Insektenfalle 24 befindlichen Insekten oder Fluginsekten 16 geeignete bzw. ausgestattete Sensorik 26 durch für elektromagnetische und/oder radioaktive Strahlung empfindliche Erfassungseinrichtungen gebildet ist oder für elektromagnetische und/oder radioaktive Strahlung empfindliche Erfassungseinrichtungen umfasst.

Die in Fig. 1 angedeuteten Lockmittel 18 können die unterschiedlichsten Lockreize 22 emittieren, so bspw. für die Insekten oder Fluginsekten 16 attraktive optische und/oder akustische und/oder sensorische Reize und/oder Wärmereize und/oder olfaktorische und/oder chemische Lockreize, mit denen die Insekten 16 jeweils anlockbar sind, wobei auch beliebige Kombinationen dieser Lockreizvarianten eingesetzt und angewendet werden können.

Der für die Aufnahme angelockter Insekten oder Fluginsekten 16 vorgesehene definierte Rauminhalt 12 bzw. der Innenraum der Insektenfalle 24 kann die aufgenommenen Insekten oder Fluginsekten 16 zumindest für ein definiertes Zeitintervall an einer Flucht hindern. Wahlweise können die Insekten 16 auch unschädlich gemacht oder vernichtet werden, nachdem sie erfasst und die erfassten Daten ausgewertet wurden.

Die Erfindung wurde unter Bezugnahme auf eine bevorzugte Ausführungsform beschrieben. Es ist jedoch für einen Fachmann vorstellbar, dass Abwandlungen oder Änderungen der Erfindung gemacht werden können, ohne dabei den Schutzbereich der nachstehenden Ansprüche zu verlassen.

### Bezugszeichenliste

- 10: System, Insektenerfassungs- und/oder Überwachungssystem, System zur
- 12: Raum, definierter Raum
- 14: Merkmal, definierbare Merkmale
- 16: Insekten, Fluginsekten, Mücken, Gelbfiebermücken
- 18: Anlockmittel, Mittel zum Anlocken von Insekten
- 20: Räumliche Umgebung, definierbare räumliche Umgebung
- 22: Lockreiz, Reize
- 24: Falle, Insektenfalle
- 26: Sensorik, Sensorik zur Erfassung von Merkmalen der Insekten
- 28: Analyse- und/oder Auswerteeinrichtung, elektronische Analyseund/oder Auswerteeinrichtung
- 30: Ausgangssignal, Sensorsignal, Sensordaten
- 32: Speichereinrichtung
- 34: Anzeigeeinrichtung, Display
- 36: Markierung, optische Markierung
- 38: Labor

## Patentansprüche

1. Verfahren zur Erfassung und/oder Überwachung von Populationen von Insekten (16), bei dem Insekten (16) wenigstens einer definierten Gattung, insbesondere Fluginsekten (16) einer definierten Gattung angelockt, zumindest temporär innerhalb eines definierten Raumes (12) festgehalten sowie in Bezug auf definierbare Merkmale (14) sensiert und/oder analysiert werden, wobei das Verfahren zumindest die folgenden Schritte vorsieht:
- Anlocken der definierten Gattung von Insekten oder Fluginsekten (16) innerhalb oder in den Bereich einer definierbaren räumlichen Umgebung (20) mittels für die Insekten oder Fluginsekten (16) attraktiver Lockreize (22), vorzugsweise durch optische und/oder akustische Reize, und/oder durch sensorische Reize und/oder Wärmereize, und/oder durch olfaktorische und/oder chemische Lockreize ausgebildete Lockreize (22),
- Überführen der angelockten Insekten oder Fluginsekten (16) in einen definierten Rauminhalt (12), insbesondere in einen Innenraum einer Insektenfalle (24) unter Verhinderung eines ungewollten Entweichens zumindest eines Großteils der in den definierten Rauminhalt (12) überführten Insekten oder Fluginsekten (16) zumindest für ein definierbares Zeitintervall,
- Erfassen spezifischer Merkmale (14) in Form von an den Insekten oder Fluginsekten (16) anhaftenden und/oder diesen eingeprägten Markierungen (36) der innerhalb des definierten Rauminhalts (12) bzw. der Insektenfalle (24) befindlichen Insekten oder Fluginsekten (16) mittels einer dafür geeigneten bzw. ausgestatteten Sensorik (26),
- Bereitstellen der mittels der Sensorik (26) erfassten spezifischen Merkmale (14) der Insekten oder Fluginsekten (16) und Übermittlung der von der Sensorik (26) gelieferten Sensorsignale (30) an eine der Sensorik (26) nachgeschaltete elektronische Analyse- und/oder Auswerteeinrichtung (28).

2. Verfahren nach Anspruch 1, bei dem als spezifische Merkmale (14) der innerhalb des definierten Rauminhalts (12) bzw. der Insektenfalle (24) befindlichen Insekten oder Fluginsekten (16) das Geschlecht und/oder die Art bzw. Gattung der Insekten erfasst wird.

3. Verfahren nach Anspruch 1 oder 2, bei dem die Markierungen (36) durch gentechnische Veränderungen an den Insekten oder Fluginsekten (16) gebildet sind, die mittels der Sensorik (26) erfasst werden.

4. Verfahren nach einem der Ansprüche 1 bis 3, bei dem die Markierungen (36) durch radioaktive Markierungen gebildet sind, die mittels der Sensorik (26) erfasst werden.

5. Verfahren nach einem der Ansprüche 1 bis 4, bei dem die Markierungen (36) durch optische und/oder fluoreszierende Markierungen (36) gebildet sind, die mittels der Sensorik (26) erfasst werden.

6. Verfahren nach einem der Ansprüche 1 bis 5, bei dem die Markierungen (36) durch den erfassten und/oder freigesetzten Populationen aufgeprägte Merkmale (14) und/oder Markierungen gebildet sind, die mittels der Sensorik (26) erfasst werden.

7. Verfahren nach einem der Ansprüche 1 bis 6, bei dem die zur Erfassung der spezifischen Merkmale (14) der innerhalb des definierten Rauminhalts (12) bzw. der Insektenfalle (24) befindlichen Insekten oder Fluginsekten (16) geeignete bzw. ausgestattete Sensorik (26) durch optische Erfassungseinrichtungen gebildet ist oder optische Erfassungseinrichtungen umfasst, wobei vorzugsweise die optischen Erfassungseinrichtungen eine der Sensorik (26) nachgeschaltete Bildauswertung umfasst.

8. Verfahren nach Anspruch 7, bei dem mittels der optischen Erfassungseinrichtungen und/oder der nachgeschalteten Bildauswertung Bewegungsmuster der sensierten Insekten oder Fluginsekten (16) erfasst und/oder der nachgeschalteten elektronischen Analyse- und/oder Auswerteeinrichtung (28) zur Verfügung gestellt werden.

9. Verfahren nach einem der Ansprüche 1 bis 8, bei dem die zur Erfassung der spezifischen Merkmale (14) der innerhalb des definierten Rauminhalts (12) bzw. der Insektenfalle (24) befindlichen Insekten oder Fluginsekten (16) geeignete bzw. ausgestattete Sensorik (26) durch akustische Erfassungseinrichtungen gebildet ist oder akustische Erfassungseinrichtungen umfasst.

10. Verfahren nach einem der Ansprüche 1 bis 9, bei dem die zur Erfassung der spezifischen Merkmale (14) der innerhalb des definierten Rauminhalts (12) bzw. der Insektenfalle (24) befindlichen Insekten oder Fluginsekten (16) geeignete bzw. ausgestattete Sensorik (26) durch für elektromagnetische und/oder radioaktive Strahlung empfindliche Erfassungseinrichtungen gebildet ist oder für elektromagnetische und/oder radioaktive Strahlung empfindliche Erfassungseinrichtungen umfasst.

11. Verfahren nach einem der Ansprüche 1 bis 10, bei dem die innerhalb der definierten räumlichen Umgebung (12) oder in der Umgebung (20) einer Insektenfalle (24) angelockten Insekten oder Fluginsekten (16) mittels für die Insekten oder Fluginsekten (16) erkennbarer und/oder attraktiver Lichtreize (22) angelockt werden.

12. Verfahren nach Anspruch 11, bei dem die optischen Lockreize (22) und/oder Lichtreize (22) im Wesentlichen durch die Aussendung von UV-Licht gebildet werden oder UV-Lichtanteile enthalten.

13. Verfahren nach einem der Ansprüche 1 bis 12, bei dem die angelockten Insekten oder Fluginsekten (16) innerhalb des definierten Rauminhalts (12) bzw. im Innenraum der Insektenfalle (24) für ein definiertes Zeitintervall an einer Flucht gehindert werden, wobei vorzugsweise die innerhalb des definierten Rauminhalts (12) bzw. im Innenraum der Insektenfalle (24) angelockten Insekten oder Fluginsekten (16) nach ihrer Erfassung wieder freigelassen werden.

14. Verfahren nach einem der Ansprüche 1 bis 12, bei dem die angelockten Insekten oder Fluginsekten (16) innerhalb des definierten Rauminhalts (12) bzw. im Innenraum der Insektenfalle (24) für unbestimmte Zeit an einer Flucht gehindert werden.

15. Verfahren nach Anspruch 13 oder 14, bei dem die innerhalb der Insektenfalle (24) befindlichen Insekten oder Fluginsekten (16) während ihres Aufenthalts in einer innerhalb der Insektenfalle (24) erzeugten Luftströmung mittels der Sensorik (26) erfasst und vermessen werden, oder bei dem die innerhalb der Insektenfalle (24) befindlichen Insekten oder Fluginsekten (16) nach ihrer Fixierung auf einer Klebefläche oder einem Netz mittels der Sensorik (26) erfasst und vermessen werden.

16. Verfahren nach einem der Ansprüche 1 bis 15, bei dem die Sensordaten (30) mindestens einer mit entsprechender Sensorik (26) ausgestatteten Insektenfalle (24) über einen definierten längeren Zeitraum erfasst und zur Analyse von Insekten- oder Fluginsektenpopulationen und/oder deren Veränderungen im Zeitverlauf erfasst, analysiert und aufbereitet werden.

17. Insektenerfassungs- und/oder Überwachungssystem (10) zur Erfassung und/oder Überwachung von angelockten und zumindest temporär innerhalb eines definierten Raumes (12) festgehaltenen sowie in Bezug auf definierbare Merkmale (14) sensierten und/oder analysierten Insekten (16) oder Fluginsekten (16) wenigstens einer definierten Gattung, wobei das System (10) zumindest umfasst:
- Mittel (18) zum Anlocken der definierten Gattung von Insekten oder Fluginsekten (16) in den Bereich oder innerhalb einer definierbaren räumlichen Umgebung (20) mittels für die Insekten oder Fluginsekten (16) attraktiver Lockreize (22), vorzugsweise durch für die Insekten oder Fluginsekten (16) attraktiver optischer und/oder akustischer und/oder sensorischer Reize (22) und/oder Wärmereize und/oder olfaktorischer und/oder chemischer Lockreize (22),
- einen definierten Rauminhalt (12), gebildet insbesondere durch einen Innenraum einer Insektenfalle (24), der/die zur Überführung und Aufnahme der angelockten Insekten oder Fluginsekten (16) unter Verhinderung eines ungewollten Entweichens zumindest eines Großteils der in den definierten Rauminhalt (12) überführten Insekten oder Fluginsekten (16) zumindest für ein definierbares Zeitintervall vorgesehen und/oder ausgestattet ist,
- eine Sensorik (26), die zur Erfassung spezifischer Merkmale (14) der innerhalb des definierten Rauminhalts (12) bzw. der Insektenfalle (24) befindlichen Insekten oder Fluginsekten (16) geeignet bzw. ausgestattet ist,
**dadurch gekennzeichnet, dass** es ich bei den spezifischen Merkmalen (14) um an den Insekten oder Fluginsekten (16) anhaftende und/oder diesen eingeprägte Markierungen (36) handelt, und dass
eine der Sensorik (26) nachgeschaltete elektronische Analyse- und/oder Auswerteeinrichtung (28) zur Erfassung und/oder Auswertung der mittels der Sensorik (26) erfassten spezifischen Merkmale (14) der Insekten oder Fluginsekten (16) vorgesehen ist.

18. System nach Anspruch 17, bei dem die zur Erfassung der spezifischen Merkmale (14) der innerhalb des definierten Rauminhalts (12) bzw. der Insektenfalle (24) befindlichen Insekten oder Fluginsekten (16) geeignete bzw. ausgestattete Sensorik (26) durch optische Erfassungseinrichtungen gebildet ist oder optische Erfassungseinrichtungen umfasst, wobei vorzugsweise die optischen Erfassungseinrichtungen eine der Sensorik (26) nachgeschaltete Bildauswertung umfasst.

19. System nach Anspruch 18, bei dem mittels der optischen Erfassungseinrichtungen und/oder der nachgeschalteten Bildauswertung Bewegungsmuster der sensierten Insekten oder Fluginsekten (16) erfasst und/oder der nachgeschalteten elektronischen Analyse- und/oder Auswerteeinrichtung (28) zur Verfügung gestellt werden.

20. System nach einem der Ansprüche 17 bis 19, bei dem die zur Erfassung der spezifischen Merkmale (14) der innerhalb des definierten Rauminhalts (12) bzw. der Insektenfalle (24) befindlichen Insekten oder Fluginsekten (16) geeignete bzw. ausgestattete Sensorik (26) durch akustische Erfassungseinrichtungen gebildet ist oder akustische Erfassungseinrichtungen umfasst.

21. System nach einem der Ansprüche 17 bis 20, bei dem die zur Erfassung der spezifischen Merkmale (14) der innerhalb des definierten Rauminhalts (12) bzw. der Insektenfalle (24) befindlichen Insekten oder Fluginsekten (16) geeignete bzw. ausgestattete Sensorik (26) durch für elektromagnetische und/oder radioaktive Strahlung empfindliche Erfassungseinrichtungen gebildet ist oder für elektromagnetische und/oder radioaktive Strahlung empfindliche Erfassungseinrichtungen umfasst.

22. System nach einem der Ansprüche 17 bis 21, bei dem die optischen Lockreize (22) und/oder Lichtreize (22) im Wesentlichen durch die Aussendung von UV-Licht gebildet sind oder UV-Lichtanteile enthalten.

23. System nach einem der Ansprüche 17 bis 22, bei dem der für die Aufnahme angelockter Insekten oder Fluginsekten (16) vorgesehene definierte Rauminhalt (12) bzw. der Innenraum der Insektenfalle (24) die aufgenommenen Insekten oder Fluginsekten (16) zumindest für ein definiertes Zeitintervall an einer Flucht hindert, wobei vorzugsweise die Insektenfalle (24) mit einer Klebefläche und/oder einem Netz zur Fixierung der gefangenen Insekten oder Fluginsekten (16) im Erfassungsbereich der Sensorik (26) ausgestattet ist.

24. System nach einem der Ansprüche 17 bis 23, das zur Durchführung eines Verfahrens gemäß einem der Ansprüche 1 bis 16 verwendbar ist oder eingesetzt wird.

## Claims

1. A method used for the detection and/or monitoring of insect populations (16), in which insects (16) of at least one defined genus, in particular flying insects (16) of a defined genus, are attracted, are at least temporarily retained within a defined compartment (12), and are sensed and/or analyzed with regard to definable features (14), wherein the method provides at least the following steps:
- attracting the defined genus of insects or flying insects (16) within or into the range of a definable spatial vicinity (20) by means of attracting stimuli (22) that are attractive to the insects or flying insects (16), preferentially by optical and/or acoustical stimuli and/or sensory stimuli and/or heat stimuli and/or by attracting stimuli (22) formed as olfactory and/or chemical attracting stimuli,
- transferring the attracted insects or flying insects (16) into a defined compartment interior (12), in particular into an interior space of an insect trap (24), while preventing at least for a definable time interval an inadvertent escape at least of a large number of the insects or flying insects (16) that have been transferred into the defined compartment interior (12);
- detecting specific features (14) of the insects or flying insects (16) located within the defined compartment interior (12) or within the insect trap (24), as appropriate, by means of a sensor technology (26) suitable or equipped therefor, as appropriate; which specific features are formed by markers (36) adhering to the insects or flying insects (16) and/or having been impressed onto them;
- providing the specific features (14) of the insects or flying insects (16) detected by means of the sensor technology (26), and transmitting the sensor signals (30) supplied by the sensor technology (26) to an electronic analysis device and/or evaluation device (28) disposed downstream from the sensor technology (26).

2. The method according to claim 1, in which the sex and/or the species or genus, as appropriate, of the insects is/are detected as specific features (14) of the insects or flying insects (16) located within the defined space (12) or within the insect trap (24), as appropriate.

3. The method according to claim 1 or 2, in which the markers (36) are formed by genetic modifications to the insects or flying insects (16), which genetic modifications are detected by means of the sensor technology (26).

4. The method according to one of the claims 1 to 3, in which the markers (36) are formed by radioactive markers, which are detected by means of the sensor technology (26).

5. The method according to one of the claims 1 to 4, in which the markers (36) are formed by optical and/or fluorescent markers, which are detected by means of the sensor technology (26).

6. The method according to one of the claims 1 to 5, in which the markers (36) are formed by features (14) and/or markers impressed onto the detected and/or released populations, which features (14) and/or markers are detected by means of the sensor technology (26).

7. The method according to one of the claims 1 to 6, in which the sensor technology (26), which is suitable or equipped, as appropriate, for the detection of the specific features (14) of the insects or flying insects (16) located within the defined compartment interior (12) or within the insect trap (24), as appropriate, is formed by optical detection devices or comprises optical detection devices, in which the optical detection devices preferentially comprise an image evaluation disposed downstream from the sensor technology (26).

8. The method according to claim 7, in which movement patterns of the sensed insects or flying insects (16) are detected and/or provided to the downstream electronic analysis device and/or evaluation device (28) by means of the optical detection devices and/or by means of the downstream image evaluation.

9. The method according to one of the claims 1 to 8, in which the sensor technology (26), which is suitable or equipped, as appropriate, for the detection of the specific features (14) of the insects or flying insects (16) located within the defined compartment interior (12) or within the insect trap (24), as appropriate, is formed by acoustic detection devices or comprises acoustic detection devices.

10. The method according to one of the claims 1 to 9, in which the sensor technology (26), which is suitable or equipped, as appropriate, for the detection of the specific features (14) of the insects or flying insects (16) located within the defined compartment interior (12) or within the insect trap (24), as appropriate, is formed by detection devices that are sensitive to electromagnetic and/or radioactive radiation or comprises detection devices that are sensitive to electromagnetic and/or radioactive radiation.

11. The method according to one of the claims 1 to 10, in which the insects or flying insects (16) attracted within the defined spatial vicinity or in the vicinity (20) of an insect trap (24) are attracted by means of light stimuli (22) that are detectable and/or attractive to the insects or flying insects (16).

12. The method according to 11, in which the optical attracting stimuli (22) and/or light stimuli (22) are substantially formed by the emission of UV light or contain UV light components.

13. The method according to one of the claims 1 to 12, in which the attracted insects or flying insects (16) within the defined compartment interior (12) or in the interior space of the insect trap (24), as appropriate, are prevented from escape for a defined time interval, wherein preferentially the insects or flying insects (16) attracted within the defined compartment interior (12) or in the interior space of the insect trap (24), as appropriate, are released again after having been detected.

14. The method according to one of the claims 1 to 12, in which the attracted insects or flying insects (16) within the defined compartment interior (12) or in the interior space of the insect trap (24), as appropriate, are prevented from escape for an undefined time.

15. The method according to claim 13 or 14, in which the insects or flying insects (16) located within the insect trap (24) are detected and measured by means of the sensor technology (26) during their stay in an airflow generated within the insect trap (24), or in which the insects or flying insects (16) located within the insect trap (24) are detected and measured by means of the sensor technology (26) after having been fixated on an adhesive surface or on a net.

16. The method according to one of the claims 1 to 15, in which the sensor data (30) from at least one insect trap (24) equipped with appropriate sensor technology (26) are detected over a defined, longer period and are detected, analyzed, and prepared for the analysis of populations of insects or flying insects and/or of their changes over the course of time.

17. An insect detection system and/or insect monitoring system (10) used for the detection and/or monitoring of insects (16) or flying insects (16) of at least one defined genus, which insects (16) or flying insects (16) are attracted and are at least temporarily retained within a defined compartment interior (12), and which are sensed and/or analyzed with regard to definable features (14), wherein the system (10) comprises at least:
- agents (18) for the attraction of the defined genus of insects or flying insects (16) into the range of or within a definable spatial vicinity (20) by means of attracting stimuli (22) that are attractive to the insects or flying insects (16); preferentially by optical and/or acoustical and/or sensory stimuli (22) and/or heat stimuli and or olfactory and/or chemical attracting stimuli (22) attractive to the insects or flying insects (16),
- a defined compartment interior (12), formed, in particular, by an interior space of an insect trap (24), which is provided and/or equipped for the transfer and accommodation of the attracted insects or flying insects (16), while preventing, at least for a definable time interval, an inadvertent escape, at least of a large number of the insects or flying insects (16) that have been transferred into the defined compartment interior (12);
- a sensor technology (26), which is suitable or equipped, as appropriate, for the detection of specific features (14) of the insects or flying insects (16) located within the defined compartment interior (12) or within the insect trap (24), as appropriate, **characterized in that** the specific features (14) are markers (36) adhering to the insects or flying insects (16) and/or having been impressed onto them, and **in that** an electronic analysis device and/or evaluation device (28) is provided, which is disposed downstream from the sensor technology (26), for the detection and/or evaluation of the specific features (14) of the insects or flying insects (16) detected by means of the sensor technology (26).

18. The system according to claim 17, in which the sensor technology (26), which is suitable or equipped, as appropriate, for the detection of the specific features (14) of the insects or flying insects (16) located within the defined compartment interior (12) or within the insect trap (24), as appropriate, is formed by optical detection devices or comprises optical detection devices, which optical detection devices preferentially comprise an image evaluation disposed downstream from the sensor technology (26).

19. The system according to claim 18, in which movement patterns of the sensed insects or flying insects (16) are detected and/or provided to the downstream electronic analysis device and/or evaluation device (28) by means of the optical detection devices and/or by means of the downstream image evaluation.

20. The system according to one of the claims 17 to 19, in which the sensor technology (26), which is suitable or equipped, as appropriate, for the detection of the specific features (14) of the insects or flying insects (16) located within the defined compartment interior (12) or within the insect trap (24), as appropriate, is formed by acoustic detection devices or comprises acoustic detection devices.

21. The system according to one of the claims 17 to 20, in which the sensor technology (26), which is suitable or equipped, as appropriate, for the detection of the specific features (14) of the insects or flying insects (16) located within the defined compartment interior (12) or within the insect trap (24), as appropriate, is formed by detection devices that are sensitive to electromagnetic and/or radioactive radiation or comprises detection devices that are sensitive to electromagnetic and/or radioactive radiation.

22. The system according to one of the claims 17 to 21, in which the optical attracting stimuli (22) and/or light stimuli (22) are substantially formed by the emission of UV light or contain UV light components.

23. The system according to one of the claims 17 to 22, in which the defined compartment interior (12) or the interior space of the insect trap (24), as appropriate, which is provided for the accommodation of attracted insects or flying insects (16), prevents the accommodated insects or flying insects (16) at least for a defined time interval from an escape, the insect trap (24) being preferentially equipped with an adhesive surface and/or with a net for the fixation of the captured insects or flying insects (16) in the detection range of the sensor technology (26).

24. The system according to one of the claims 17 to 23, which is usable or employed to carry out a method according to one of the claims 1 to 16.

## Revendications

1. Procédé de détection et/ou de surveillance de populations d'insectes (16), dans lequel des insectes (16) d'au moins un genre défini, en particulier des insectes volants (16) d'un genre défini, sont attirés, sont retenus au moins temporairement à l'intérieur d'un espace défini (12), ainsi que détectés et/ou analysés en ce qui concerne des caractéristiques définissables (14), le procédé prévoyant au moins les étapes suivantes consistant à:
- attirer le genre défini d'insectes ou d'insectes volants (16) à l'intérieur ou dans la zone d'un environnement spatial définissable (20) au moyen de stimuli attirants (22) attractifs pour les insectes ou insectes volants (16), de préférence par des stimuli optiques et/ou acoustiques, et/ou par des stimuli sensoriels et/ou des stimuli thermiques, et/ou par des stimuli attirants (22) formés par des stimuli attirants olfactifs et/ou chimiques,
- transférer les insectes ou insectes volants (16) attirés dans un volume défini (12), en particulier dans un espace intérieur d'un piège à insectes (24) tout en empêchant une fuite non voulue d'au moins une grande partie des insectes ou insectes volants (16) transférés dans le volume défini (12), au moins pour un intervalle de temps définissable,
- détecter, au moyen d'un système de capteurs (26) adapté ou bien équipé à cette fin, des caractéristiques spécifiques (14) des insectes ou insectes volants (16) se trouvant à l'intérieur du volume défini (12) ou bien du piège à insectes (24), sous forme de marquages (36) adhérant et/ou gravés sur les insectes ou insectes volants (16),
- fournir les caractéristiques spécifiques (14) des insectes ou insectes volants (16), détectées au moyen du système de capteurs (26), et transmettre les signaux de capteur (30) fournis par le système de capteurs (26) à un dispositif électronique d'analyse et/ou dévaluation (28) qui est monté en aval du système de capteurs (26).

2. Procédé selon la revendication 1, dans lequel on détecte le sexe et/ou le type ou bien le genre des insectes en tant que caractéristiques spécifiques (14) des insectes ou insectes volants (16) se trouvant à l'intérieur du volume défini (12) ou bien du piège à insectes (24).

3. Procédé selon la revendication 1 ou 2, dans lequel les marquages (36) sont formés par des modifications de génie génétique sur les insectes ou insectes volants (16) qui sont détectées au moyen du système de capteurs (26).

4. Procédé selon l'une quelconque des revendications 1 à 3, dans lequel les marquages (36) sont formés par des marquages radioactifs qui sont détectés au moyen du système de capteurs (26).

5. Procédé selon l'une quelconque des revendications 1 à 4, dans lequel les marquages (36) sont formés par des marquages optiques et/ou fluorescents qui sont détectés au moyen du système de capteurs (26).

6. Procédé selon l'une quelconque des revendications 1 à 5, dans lequel les marquages (36) sont formés par des caractéristiques (14) et/ou des marquages qui sont gravé(e)s sur les populations détectées et/ou libérées et qui sont détecté(e)s au moyen du système de capteurs (26).

7. Procédé selon l'une quelconque des revendications 1 à 6, dans lequel le système de capteurs (16) adapté ou bien équipé pour détecter les caractéristiques spécifiques (14) des insectes ou insectes volants (16) se trouvant à l'intérieur du volume défini (12) ou bien du piège à insectes (24) est formé par des dispositifs de détection optique ou comprend des dispositifs de détection optique, dans lequel, de préférence, les dispositifs de détection optique comprennent un système d'évaluation d'image monté en aval du système de capteurs (26).

8. Procédé selon la revendication 7, dans lequel des modèles de mouvement des insectes ou insectes volants (16) détectés sont détectés au moyen des dispositifs de détection optique et/ou du système d'évaluation d'image monté en aval et/ou sont fournis au dispositif électronique d'analyse et/ou d'évaluation (28) monté en aval.

9. Procédé selon l'une quelconque des revendications 1 à 8, dans lequel le système de capteurs (26) adapté ou bien équipé pour détecter les caractéristiques spécifiques (14) des insectes ou insectes volants (16) se trouvant à l'intérieur du volume défini (12) ou bien du piège à insectes (24) est formé par des dispositifs de détection acoustique ou comprend des dispositifs de détection acoustique.

10. Procédé selon l'une quelconque des revendications 1 à 9, dans lequel le système de capteurs (26) adapté ou bien équipé pour détecter les caractéristiques spécifiques (14) des insectes ou insectes volants (16) se trouvant à l'intérieur du volume défini (12) ou bien du piège à insectes (24) est formé par des dispositifs de détection sensibles au rayonnement électromagnétique et/ou radioactif ou comprend des dispositifs de détection sensibles au rayonnement électromagnétique et/ou radioactif.

11. Procédé selon l'une quelconque des revendications 1 à 10, dans lequel les insectes ou les insectes volants (16) attirés à l'intérieur de l'environnement spatial défini (12) ou dans l'environnement (20) d'un piège à insectes (24) sont attirés au moyen de stimuli lumineux (22) reconnaissables et/ou attractifs pour les insectes ou les insectes volants (16).

12. Procédé selon la revendication 11, dans lequel les stimuli attirants optiques (22) et/ou les stimuli lumineux (22) sont formés pour l'essentiel par l'émission de lumière UV ou contiennent des composants de lumière UV.

13. Procédé selon l'une quelconque des revendications 1 à 12, dans lequel les insectes ou insectes volants (16) attirés à l'intérieur du volume défini (12) ou bien à l'intérieur du piège à insectes (24) sont empêchés de s'échapper pendant un intervalle de temps défini, dans lequel, de préférence, les insectes ou insectes volants (16) attirés à l'intérieur du volume défini (12) ou bien à l'intérieur du piège à insectes (24) sont à nouveau libérés après avoir été détectés.

14. Procédé selon l'une quelconque des revendications 1 à 12, dans lequel les insectes ou insectes volants (16) attirés à l'intérieur du volume défini (12) ou bien à l'intérieur du piège à insectes (24) sont empêchés de s'échapper pendant une durée indéterminée.

15. Procédé selon la revendication 13 ou 14, dans lequel les insectes ou insectes volants (16) se trouvant à l'intérieur du piège à insectes (24) sont détectés et mesurés au moyen du système de capteur (26), pendant leur séjour, dans un flux d'air généré à l'intérieur du piège à insectes (24), ou dans lequel les insectes ou insectes volants (16) se trouvant à l'intérieur du piège à insectes (24) sont détectés et mesurés au moyen du système de capteurs (26) après avoir été fixés à une surface adhésive ou à un filet.

16. Procédé selon l'une quelconque des revendications 1 à 15, dans lequel les données de capteurs (30) d'au moins un piège à insectes (24) équipé d'un système de capteurs (26) correspondant sont saisies sur une durée plus longue définie et sont enregistrées, analysées et traitées pour analyser des populations d'insectes ou d'insectes volants et/ou leurs changements dans le temps.

17. Système de détection et/ou de surveillance d'insectes (10) destiné à détecter et/ou à surveiller des insectes (16) ou insectes volants (16) d'au moins un genre défini, qui sont attirés et retenus au moins temporairement à l'intérieur d'un espace défini (12) et détectés et/ou analysés en ce qui concerne des caractéristiques définissables (14), dans lequel le système (10) comprend au moins:
- des moyens (18) pour attirer le genre défini d'insectes ou d'insectes volants (16) dans la zone ou à l'intérieur d'un environnement spatial définissable (20) au moyen de stimuli attirants (22) attractifs pour les insectes ou insectes volants (16), de préférence par des stimuli (22) optiques et/ou acoustiques et/ou sensoriels et/ou des stimuli thermiques et/ou des stimuli attirants (22) olfactifs et/ou chimiques qui sont attractifs pour les insectes ou insectes volants (16),
- un volume défini (12), formé en particulier par un espace intérieur d'un piège à insectes (24), qui est prévu et/ou équipé pour transférer et recevoir les insectes ou insectes volants (16) attirés tout en empêchant une fuite non voulue d'au moins une grande partie des insectes ou insectes volants (16) transférés dans le volume défini (12), au moins pour un intervalle de temps définissable,
- un système de capteurs (16) qui est adapté ou bien équipé pour détecter des caractéristiques spécifiques (14) des insectes ou insectes volants (16) se trouvant à l'intérieur du volume défini (12) ou bien du piège à insectes (24),
**caractérisé par le fait que** les caractéristiques spécifiques (14) sont des marquages (36) adhérents et/ou gravées sur les insectes ou insectes volants (16), et qu' un dispositif électronique d'analyse et/ou d'évaluation (28) monté en aval du système de capteurs (26) et destiné à détecter et/ou à évaluer les caractéristiques spécifiques (14) des insectes ou insectes volants (16) détectées au moyen du système de capteurs (26) est prévu.

18. Système selon la revendication 17, dans lequel le système de capteurs (26) adapté ou bien équipé pour détecter les caractéristiques spécifiques (14) des insectes ou insectes volants (16) se trouvant à l'intérieur du volume défini (12) ou bien du piège à insectes (24) est formé par des dispositifs de détection optique ou comprend des dispositifs de détection optique, dans lequel, de préférence, les dispositifs de détection optique comprennent un système d'évaluation d'image monté en aval du système de capteurs (26).

19. Système selon la revendication 18 dans lequel des modèles de mouvement des insectes ou insectes volants (16) détectés sont détectés au moyen des dispositifs de détection optique et/ou du système d'évaluation d'image monté en aval et/ou sont fournis au dispositif électronique d'analyse et/ou d'évaluation (28) monté en aval.

20. Système selon l'une quelconque des revendications 17 à 19, dans lequel le système de capteurs (26) adapté ou bien équipé pour détecter les caractéristiques spécifiques (14) des insectes ou insectes volants (16) se trouvant à l'intérieur du volume défini (12) ou bien du piège à insectes (24) est formé par des dispositifs de détection acoustique ou comprend des dispositifs de détection acoustique.

21. Système selon l'une quelconque des revendications 17 à 20, dans lequel le système de capteurs (26) adapté ou bien équipé pour détecter les caractéristiques spécifiques (14) des insectes ou insectes volants (16) se trouvant à l'intérieur du volume défini (12) ou bien du piège à insectes (24) est formé par des dispositifs de détection sensibles au rayonnement électromagnétique et/ou radioactif ou comprend des dispositifs de détection sensibles au rayonnement électromagnétique et/ou radioactif.

22. Système selon l'une quelconque des revendications 17 à 21, dans lequel les stimuli attirants optiques (22) et/ou les stimuli lumineux (22) sont formés pour l'essentiel par l'émission de lumière UV ou contiennent des composants de lumière UV.

23. Système selon l'une quelconque des revendications 17 à 22, dans lequel le volume défini (12) ou bien l'intérieur du piège à insectes (24) prévu pour recevoir des insectes ou insectes volants (16) attirés empêche les insectes ou insectes volants (16) reçus, au moins pour un intervalle de temps défini, de s'échapper, dans lequel, de préférence, le piège à insectes (24) est équipé d'une surface adhésive et/ou d'un filet pour fixer les insectes ou insectes volants (16) capturés dans la zone de détection du système de capteurs (26).

24. Système selon l'une quelconque des revendications 17 à 23, qui peut être utilisé ou est employé pour mettre en œuvre un procédé selon l'une quelconque des revendications 1 à 16.
